# EUROPEAN PATENT APPLICATION

(11) **EP 1 439 171 A1**
(43) Date of publication of application: **21.07.2004**
(21) Application number: 02800719.3
(22) Date of filing: 01.10.2002
(51) Int. Cl.: C07D 277/30

(54) **PROCESS FOR PREPARATION OF ETHENE DERIVATIVES**

(30) Priority: 02.10.2001 JP 2001306243
(71) Applicant: NIPPON SODA CO., LTD., Chiyoda-ku, Tokyo 100-8165 (JP)
(72) Inventor: MATSUI, N., Takaoka Plant, Nippon Soda Co., Ltd, Takaoka-shi, Toyama 933-8507 (JP); FURUKAWA, H., Odawara Research Center, Odawara-shi, Kanagawa 250-0280 (JP); OGIHARA, Atsushi, Takaoka Plant, Takaoka-shi, Toyama 933-8507 (JP); KIMURA, Yasuharu, c/o Takaoka Plant, Takaoka-shi, Toyama 933-8507 (JP)
(74) Representative: Wibbelmann, Jobst, Dr., Dipl.-Chem.
(86) International application number: PCT/JP2002/010207
(87) International publication number: WO 2003/031421

(57) **Abstract**

The invention aims at providing a process by which ethene derivatives of enol form represented by the general formula (I) can be obtained in high yield with the configuration of enolic double bond being retained: (I) [wherein E is C(=X)R4, S(O)nR4, P(=X)R4R5, or the like] and which comprises reacting a compound represented by the general formula (II): (II) with a compound represented by the general formula (III): EY ...(III) [wherein E is as defined above; and Y is chloro, bromo, or CN] in the presence of a base and a pyridine derivative represented by the general formula (IV): (IV) [wherein R6 is alkyl or the like].

## Description

### Field of Invention:

The present invention relates to processes for the preparation of ethene derivatives of enol form that is a partial structure useful when the derivatives are used as intermediates for producing agrochemicals and drugs. In more detail, it relates to processes for producing enol O-acyl compounds and the like and stereoselective processes for producing them.

### Background Art:

A process for the preparation of enol O-acyl compounds and the like is disclosed, for example, in Japanese Patent Laid-open No. 2001-106665 that an enol compound is reacted with a thiocarbonyl chloride (R'X') or the like in an organic solvent with an alkali metal, alkali metal carbonate, alkali metal hydride or tertiary amine as a base, according to the reaction scheme shown below. 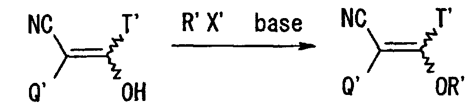 (wherein, Q' and T' are each independently optionally substituted phenyl or heterocyclic group, or the like).

A method according to the following reaction scheme is disclosed in Japanese Patent Laid-open No. Sho 55-154962. 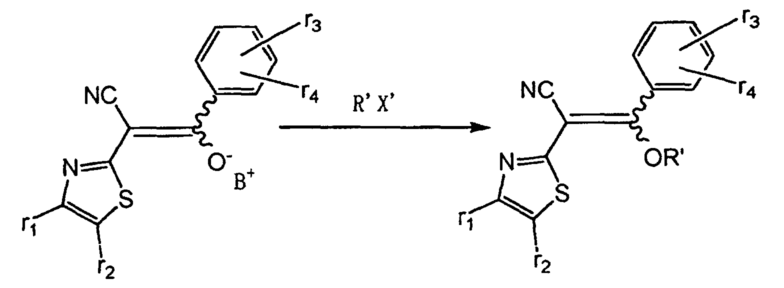 (wherein, B⁺ is an ammonium salt or an alkali metal cation).

In either case, however, the yields are not satisfactory, and the reactions do not always proceed selectively when the yields are taken into account despite of the descriptions of stereoselective productions of the products.

As for reactions similar to those of the present invention, a process for the preparation of O-acyl compounds of cyclohexan-1,3-dione enols according to the following reaction scheme is disclosed in Japanese Patent Laid-open No. Sho 54-27547.

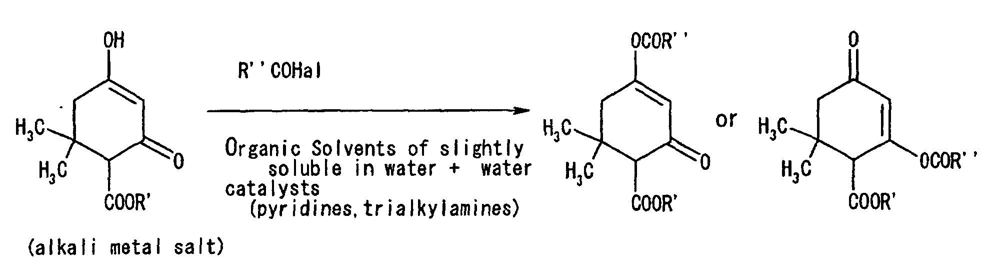

There exist, however, no stereoisomers of these compounds with regard to the enolic double bond, as it is obvious from the above reaction equation.

It is an object of the present invention to provide a process for the preparation of an ethene derivative of enol form in high yield and for producing an ethene derivative by retaining the configuration of the enolic double bond

The inventors studied in earnest to achieve the above object. As a result it was found that the object was achieved by selections of appropriate types of base and catalyst for use and further by adjustments of reaction methods. Thus, the present invention has been completed.

### Disclosure of the Invention:

The present invention relates to a process for the preparation of an ethene derivative represented by Formula (I) 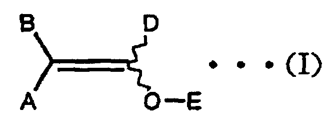 (wherein, A is optionally substituted hydrocarbon having 1 to 20 carbons, optionally substituted heterocyclic group, or a group represented by Formula R¹O, R¹S or R¹₁H₂₋₁N; R¹ is optionally substituted hydrocarbon having 1 to 20 carbons or optionally substituted heterocyclic group; I is 1 or 2; and when 1 is 2, R¹ may be the same or different; B is optionally substituted hydrocarbon having 1 to 20 carbons, optionally substituted heterocyclic group, CN, isonitrile, NO₂, N₃, CHO, or a group represented by Formula C(=X)R², S(O)mR² or P(=X)R²R³; R² and R³ are each independently optionally substituted heterocyclic group, optionally substituted hydrocarbon having 1 to 20 carbons, or a group represented by Formula R²⁰O, R²⁰S or R²⁰ₖH₂₋ₖN; R²⁰ is optionally substituted hydrocarbon having 1 to 20 carbons, or optionally substituted heterocyclic group; X is oxygen, sulfur, selenium, or a group represented by Formula NR²¹; R²¹ is optionally substituted hydrocarbon having 1 to 20 carbons, optionally substituted heterocyclic group, hydroxyl, or a group represented by Formula R²²O or R²²ₜH₂₋ₜN; R²² is optionally substituted hydrocarbon having 1 to 20 carbons, or optionally substituted heterocyclic group; m is 0, 1 or 2; k is 1 or 2; and when k is 2, R²⁰ may be the same or different; t is 1 or 2; and when t is 2, R²² may be the same or different; D is optionally substituted hydrocarbon having 1 to 20 carbons, or optionally substituted heterocyclic group; E is a group represented by Formula C(=X)R⁴, S(O)nR⁴ or P(=X)R⁴R⁵, alkoxymethyl having 1 to 6 carbons, alkylcarbonyloxymethyl having 1 to 6 carbons, cycloalkylcarbonyloxymethyl having 3 to 6 carbons, alkoxycarbonyloxymethyl having 1 to 6 carbons, optionally substituted phenylcarbonyloxymethyl, alkylthiomethyl having 1 to 6 carbons, alkylcarbonylthiomethyl having 1 to 6 carbons, cycloalkylcarbonylthiomethyl having 3 to 6 carbons, alkoxycarbonylthiomethyl having 1 to 6 carbons, optionally substituted phenylcarbonylthiomethyl, or optionally substituted phenylmethyl; R⁴ and R⁵ are each independently optionally substituted heterocyclic group, optionally substituted hydrocarbon having 1 to 20 carbons, or a group represented by Formula R⁴⁰O, R⁴⁰S or R⁴⁰_{q}H_{2-q}N; R⁴⁰ is optionally substituted hydrocarbon having 1 to 20 carbons, or optionally substituted heterocyclic group; X is oxygen, sulfur, selenium, or a group represented by Formula NR⁵¹; R⁵¹ is optionally substituted hydrocarbon having 1 to 20 carbons, or hydroxyl; n is 0, 1 or 2; q is 1 or 2; when q is 2, R⁴⁰ may be the same or different), characterized in that a compound represented by Formula (II) 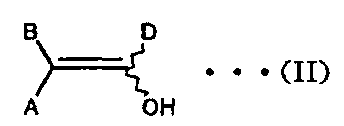 (wherein, A, B and D are as defined above) is reacted with a compound represented by Formula (III)

EY (III)

(wherein, E is as defined above; and Y is chlorine, bromine or CN; however, Y is chlorine or bromine when E is alkoxymethyl having 1 to 6 carbons, alkylcarbonyloxymethyl having 1 to 6 carbons, cycloalkylcarbonyloxymethyl having 3 to 6 carbons, alkoxycarbonyloxymethyl having 1 to 6 carbons, optionally substituted phenylcarbonyloxymethyl, alkylthiomethyl having 1 to 6 carbons, alkylcarbonylthiomethyl having 1 to 6 carbons, cycloalkylcarbonylthiomethyl having 3 to 6 carbons, alkoxycarbonylthiomethyl having 1 to 6 carbons, optionally substituted phenylcarbonylthiomethyl, or optionally substituted phenylmethyl) in the presence of a base and a pyridine derivative represented by Formula (IV) 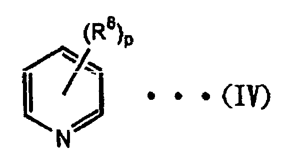 (wherein, R⁶ is hydrocarbon having 1 to 9 carbons; p is 0 or an integer of 1 to 3; and when p is 2 or more, R⁶ may be the same or different).

Examples of substituents represented by E, A and D are shown in the following.
E: alkylcarbonyl having 1 to 10 carbons, alkylthiocarbonyl or (alkylthio)carbonyl;
A: optionally substituted phenyl, benzyl or aromatic heterocyclic group;
D: optionally substituted phenyl or pyrazolyl.

Their actual examples are shown below:
A: 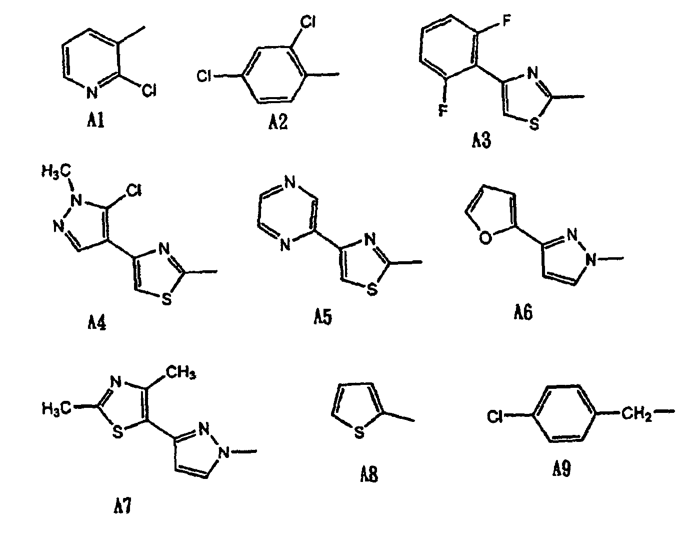
D: 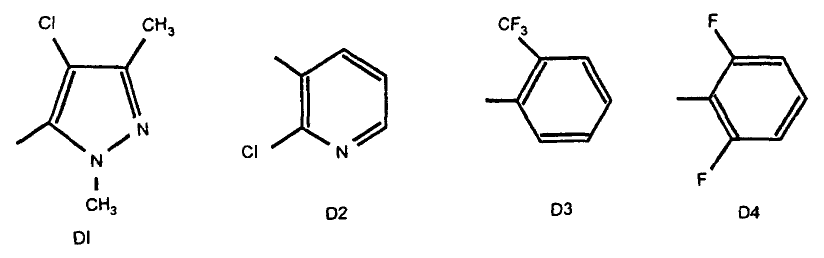 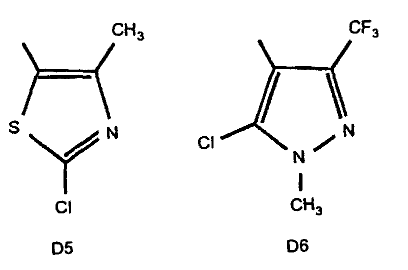
E:

| | | | | | |
|---|---|---|---|---|---|
| E1 | -COCH₃ | E2 | -COC(CH₃)₃ | E3 | -COSCH₃ |
| E4 | -COC(CH₃)₂-C₂H₅ | E5 | -COC(CH₃)₂-C₃H₇ | E6 | -CSCH₃ |

The present invention is particularly useful for producing compounds where B is a CN group.

In the compounds of Formula (II), Y is chlorine, bromine or CN. When Y is CN, reactions of some of the compounds may not proceed or target compounds not be produced, depending on a group of E. In the above definitions, examples of substituents of the optionally substituted groups, such as hydrocarbon, phenyl and heterocyclic, include alkyl having 1 to 4 carbons, halogen, alkoxy having 1 to 4 carbons, or haloalkyl having 1 to 4 carbons.

A reaction is carried out in an organic solvent or a heterogeneous system of water and an organic solvent.

In case that a reaction is performed in an organic solvent, examples of processes include that [1] a compound of Formula (III) and a base are added in sequence to an organic solvent containing a compound of Formula (II); [2] a compound of Formula (II) and a compound of Formula (III) are added one by one to an organic solvent containing a base: [3] a mixture of a compound of Formula (II) and a compound of Formula (III) is added to an organic solvent containing a base; [4] a compound of Formula (III) and a base are added simultaneously to an organic solvent containing a compound of Formula (II); [5] a compound of Formula (II) and a compound of Formula (III) are added at the same time to an organic solvent containing a base; and [6] a compound of Formula (III) is added to a solution containing a compound of Formula (II) and a tertiary amine, and further a heterocyclic compound containing nitrogen is added Each compound can be mixed with a solvent or added to it by any method. Usable methods are that one component is gradually dropped into the other, all ingredients are added together, or a component is added little by little over a few times, within a range of the reaction conditions such as temperature.

A reaction temperature is between -10 to 50°C, preferably 30°C or lower. If a reaction is carried out at 50°C or higher, a ratio of isomers tends to decrease. If below -10°C, there is a tendency of a slow reaction rate and low yield. Any base can be used. Preferred is a tertiary amine.

Actual examples of usable tertiary amines include 1,8-diazabicyclo[5.4.0]undec-7ene, 1,5-diazabicyclo[4.3.0]non-5-ene, 6-dibutylamino-1,8-diazabicyclo[5.4.0]undec-7-ene, triethylenediamine, N,N-dimethylaminopyridine, trimethylamine, triethylamine, tri-n-butylamine, N,N-dimethylcyclohexylamine, N,N-diethylaniline, quinoline and diisopropylethylamine. These can be uses alone or as a mixture of two or more. An amount of a base used depends on how much a pyridine derivative of Formula (IV) is used. A total amount of a base and a pyridine derivative to a volume of a compound of Formula (II) is preferably an equivalent or more, more preferably between 1.05 and 1.50 equivalents, in mole ratio. If less than 1.05 equivalents, the reaction does not complete so that starting materials remain unreacted. If more than 1.50 equivalents, the occurrence of hydrolysis of a compound of Formula (III) may result in an incompletion of the reaction.

For the pyridine derivatives of Formula (IV) that are used in the present invention, R⁶ is hydrocarbon having 1 to 9 carbons, and n is 0 or an integer of 1 to 3. When n is 2 or more, R⁶ may be the same or different. Actual examples of R⁶ include methyl, ethyl, n-propyl, isopropyl, n-butyl, s-butyl, t-butyl and n-pentyl. As for the pyridine derivatives of Formula (IV), pyridine, α-picoline, β-picoline, γ-picoline, 3,5-lutidine, 2,4-lutidine, s-collidine, γ-collidine and 4-benzylpyridine are actually exemplified.

A pyridine derivative is preferably used at a catalytic amount to an amount of a compound of Formula (II). "A catalytic amount" refers to an equivalent mole or less to an amount of a compound of Formula (II). It is favorable to use a pyridine derivative in a range of 0.05 to 60 mole%, more favorably between 0.1 and 20 mole%. There are no restrictions on organic solvents for use, if they are inactive in the reactions, and dissolve starting materials, products and others to some extent. Their actual examples include halogen solvents such as methylene chloride, chloroform, dichloroethane and chlorobenzene; hydrocarbon solvents such as benzene, toluene, xylene, hexane and cyclohexane; ester solvents such as methyl acetate, ethyl acetate, isopropyl acetate and butyl acetate; ketone solvents such as acetone and methyl isobutyl ketone; ether solvents such as diethyl ether, nitrile solvents such as acetonitrile and benzonitrile; nitro solvents such as nitrobenzene; and DMF and DMSO. These can be used alone or as a mixture of two or more.

A more preferable embodiment to implement the present invention is that a reaction is carried out in a heterogeneous system of water and an organic solvent. In this case, usable bases are inorganic bases including alkali metal hydroxides such as sodium hydroxide and alkali metal carbonates such as potassium carbonate; or organic bases such as tertiary amines. Further, a combined use of inorganic and organic bases may improve isomer ratios in some cases. It is also possible to use an alkali metal salt of a compound of Formula (II) as a substitute of a base. The same amines as those exemplified for the reactions in organic solvents can be used as tertiary amines.

Any solvent can be used for the reaction, if it is inactive to a compound of Formula (III). Preferred are those having low solubility in water, and dissolving a compound of Formula (II) to some extent Actual examples include halogen solvents such as methylene chloride, chloroform, dichloroethane and chlorobenzene; hydrocarbon solvents such as benzene, toluene, xylene, hexane and cyclohexane; ester solvents such as methyl acetate, ethyl acetate, isopropyl acetate and butyl acetate; ketone solvents such as methyl isobutyl ketone; ether solvents such as diethyl ether, nitrile solvents such as benzonitrile; and nitro solvents such as nitrobenzene. These can be used alone or as a mixture of two or more. Solvents that have polar groups so as to be considered to have high affinity with water can be used in the reaction, if non-polar groups share a large portion in a molecule. A total amount of water and an organic solvent to use may be determined at discretion within a range that a compound of Formula (II) is dissolved or can be stirred in a reaction system. Water can be mixed with an organic solvent at any ratio. An increase of an organic solvent may sometimes improve selectivity. In the case of the reaction in the heterogeneous system, a further use of a phase-transfer catalyst may improve the reaction selectivity in some cases. Actual examples of usable phase-transfer catalysts include onium salts such as quaternary ammonium salts and quaternary phosphonium salts, and crown compounds; and, in more detail, tetrabutyl ammonium chloride, benzyltributyl ammonium chloride, tetraethyl phosphonium chloride, tetraphenyl phosphonium bromide and 18-crown-6.

An amount of a phase-transfer catalyst to use is an equivalent or less in mole ratio to a volume of a compound of Formula (II), and preferably in a range of 0.5 to 30 mole%.

Any of the following reaction methods can be adopted: [1] a compound of Formula (II) is mixed with a compound of Formula (III) in a mixed solvent of an organic solvent and water, and a base is added; [2] a compound of Formula (III) is mixed with a base in a mixed solvent of an organic solvent and water, and a compound of Formula (II) is added; [3] a compound of Formula (II) is mixed with a base in a mixed solvent of an organic solvent and water, and a compound of Formula (III) is added; [4] a mixture of a compound of Formula (II) and a compound of Formula (III) or a solution containing both of the compounds is added to an aqueous solution of a base or a mixed solution of a base, water and an organic solvent, and [5] a compound of Formula (II) and a compound of Formula (III) or a solution containing one of them and a solution containing the other are simultaneously added to an aqueous solution of a base or a mixed solution of a base, water and an organic solvent. When the stability of the compound of Formula (III) is taken into account, Method [2], [3] or [4] is preferred. Any method can be applied to mix or add individual compounds to solvents. The following methods can be adopted: one component is gradually dropped into the other, all ingredients are added together, or a component is added little by little over a few times, within a range of the reaction conditions such as temperature. A phase-transfer catalyst, if used, can be added at any time and by any means.

A reaction is performed favorably at a temperature between -10 and 50°C, more favorably 40°C or below, and most favorably 30°C or lower, throughout the course. If a reaction is carried out at 50°C or higher, the compound of Formula (II), a starting material, remains unreacted If 5°C or lower, there is a tendency that a reaction rate is slow, degradation of the compound of Formula (III) progresses, and starting materials remain unreacted. Usual post treatments after the completion of the reaction give the target compound whether the reaction is performed in an organic solvent or in a heterogeneous system of water and an organic solvent.

Representative examples of compounds produced according to the processes of the present invention are listed below. A1 to A9, D1 to D6 and E1 to E6 are as defined above.

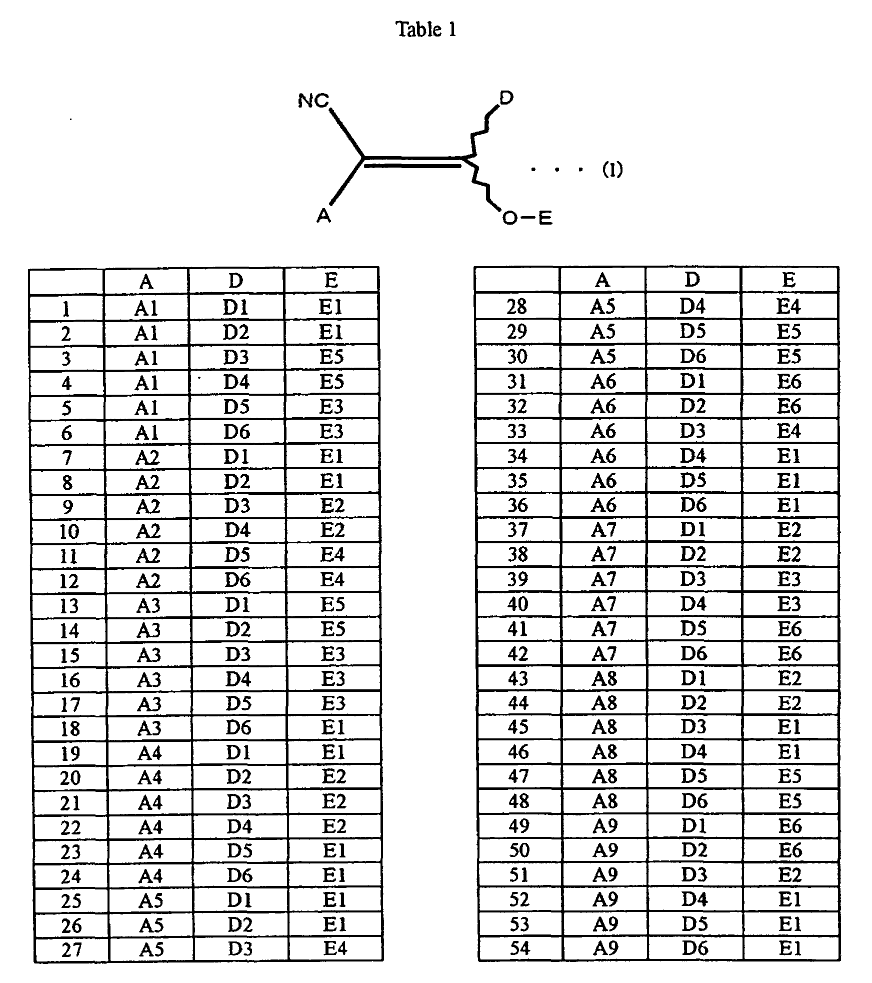

A compound of Formula (II) may exist as a single compound, or be an equilibrium mixture shown in the following equation when it is a mixture containing stereoisomers with regard to the double bond.

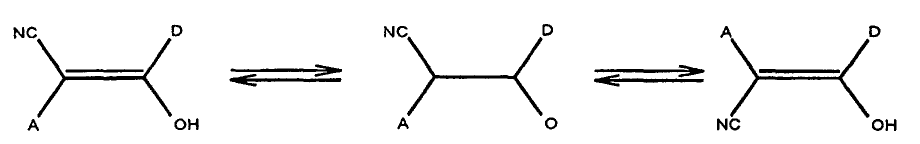

### Best Form to Implement the Invention

The present invention is described in detail in reference to Examples. The scope of the present invention is not limited to the examples.

In Examples 1 through 17 and Comparative Example 1, the target compound may be obtained as a mixture of two geometrical isomers. The main product (abbreviated as A) refers to the product with a shorter retention time in measurements by reversed-phase liquid chromatography (HPLC). The other geometrical isomer (abbreviated as B) has a longer retention time. The production ratio of A to B is represented by a ratio of two peak areas obtained by the chromatography.

### HPLC measuring conditions:

- Mobile phase: CH₃CN / H₂O / 10% H₃PO₄
770 ml / 230 ml / 1.0 ml
- Column: : Inertsil ODS-3 (GL Science Co., Ltd.)
- Column temperature: : 40°C
- Detection wavelength: : 254 nm
- Flow rate: : 1.0 ml/minute
- Retention time: : A≒20 minutes, B≒22 minutes

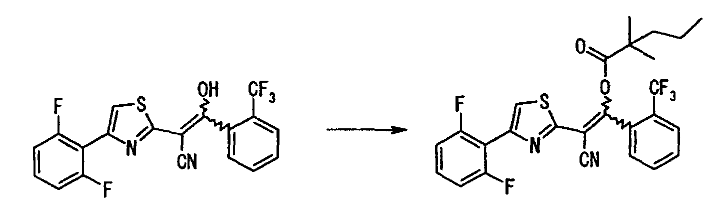

The above represents the target compound of the examples described below.

### Example 1

To a mixture of 2.5g of 2-[4-(2,6-difluorophenyl)-thiazol-2-yl]-3-hydroxy-3-(2-tnfluoromethylphenyl)-acrylonitnle, 20 ml of an aqueous solution of sodium hydroxide (containing 0.26g of NaOH) and 20 ml of toluene was added 0.13g of 3,5-lutidine, and then 1.09g of 2,2-dimethylpentanoyl chloride was dropped at 30°C. The resulting solution was stirred for an hour at the same temperature A quantitative analysis by high-performance liquid chromatography (HPLC) showed that the reaction solution contained 3.07g of the target compound. Yield: 99.3% (production ratio A:B = 95.3:4.7)

### Example 2

To a mixture of 2.5g of 2-[4-(2,6-difluorophenyl)-thiazol-2-yl]-3-hydroxy-3-(2-trifluoromethylphenyl)-acrylonitrile, 10 ml of water and 30 ml of toluene were added 0.85g of potassium carbonate and 0.26g of 3,5-lutidine, and then 1.19g of 2,2-dimethylpentanoyl chloride was dropped at 30°C. The resulting solution was stirred for an hour at the same temperature. A quantitative analysis by HPLC showed that the reaction solution contained 3.17g of the target compound. Yield: 99.6% (production ratio A:B = 93.2:6.8)

### Example 3

To a mixture of 2.5g of 2-[4-(2,6-difluorophenyl)-thiazol-2-yl]-3-hydroxy-3-(2-trifluoromethylphenyl)-acrylonitrile, 30 ml of toluene and 5 ml of water were added 0.79g of diisopropylethylamine and 0.26g of 3,5-lutidine, and then 1.19g of 2,2-dimethylpentanoyl chloride was dropped at 30°C. The resulting solution was stirred for 2 hours at the same temperature. A quantitative analysis by HPLC showed that the reaction solution contained 3. 10g of the target compound. Yield: 97.3% (production ratio A:B = 95.4:4.6)

### Example 4

To a mixture of 2.5g of 2-[4-(2,6-difluorophenyl)-thiazol-2-yl]-3-hydroxy-3-(2-trifluoromethylphenyl)-acrylonitrile, 30 ml of toluene and 5 ml of water were added 0.62g of triethylamine and 0.26g of 3,5-lutidine, and then 1.19g of 2,2-dimethylpentanoyl chloride was dropped at 30°C. The resulting solution was stirred for an hour at the same temperature. A quantitative analysis by HPLC showed that the reaction solution contained 3.17g of the target compound. Yield: 99.5% (production ratio A:B = 96.2:3.8)

### Example 5

To a mixture of 2.5g of 2-[4-(2,6-difluorophenyl)-thiazol-2-yl]-3-hydroxy-3-(2-trifluoromethylphenyl)-acrylonitrile, 15 ml of toluene and 15 ml of an aqueous solution of sodium hydroxide (containing 0.26g of NaOH) was added 0.11g of γ-picoline, and then 1.19g of 2,2-dimethylpentanoyl chloride was dropped at 30°C. The resulting solution was stirred for an hour at the same temperature. A quantitative analysis by HPLC showed that the reaction solution contained 3.17g of the target compound. Yield: 99.5% (production ratio A:B = 94.4:5.6)

### Example 6

To a mixture of 2.5g of 2-[4-(2,6-difluorophenyl)-thiazol-2-yl]-3-hydroxy-3-(2-trifluoromethylphenyl)-acrylonitrile and 20 ml of toluene were added 0.74g of triethylamine and 1.19g of 2,2-dimethylpentanoyl chloride. To the obtained mixture was dropped a solution of 0.11g of γ-picoline in 10 ml of water at 10°C. The resulting solution was stirred for an hour at 10 to 22°C. A quantitative analysis by HPLC showed that the reaction solution contained 3.18g of the target compound. Yield: 99.9% (production ratio A:B = 98.8:1.2)

### Example 7

Example 6 was repeated except that chloroform was used in place of toluene. A quantitative analysis by HPLC showed that the reaction solution contained 3.17g of the target compound. Yield: 99.5% (production ratio A:B = 99.4:0.6)

### Example 8

Example 6 was repeated except that butyl acetate was used in place of toluene. A quantitative analysis by HPLC showed that the reaction solution contained 3.18g of the target compound. Yield: 99.9% (production ratio A:B = 99.9:0.1)

### Example 9

To a mixture of 2.5g of 2-[4-(2,6-difluorophenyl)-thiazol-2-yl]-3-hydroxy-3-(2-trifluoromethylphenyl)-acrylonitrile, 8 ml of toluene and 15 ml of water was added 1.19g of 2,2-dimethylpentanoyl chloride, and then 7 ml of a toluene solution containing 0.74g of triethylamine and 0.11g of γ-picoline was dropped at 25°C over 25 minutes. The resulting solution was further stirred for 30 minutes at 25 to 30°C. A quantitative analysis by HPLC showed that the reaction solution contained 3.18g of the target compound. Yield: 99.7% (production ratio A:B = 89:11)

### Example 10

To a mixture of 1.98g of 2-[4-(2,6-difluorophenyl)-thiazol-2-yl]-3-hydroxy-3-(2-trifluoromethylphenyl)-acrylonitrile and 15 ml of chlorofrm were added 0.59g of triethylamine and 0.94g of 2,2-dimethylpentanoyl chloride. To the obtained mixture was added 0.09g of γ-picoline at 10 to 15°C. The resulting solution was stirred for 5 minutes at 10 to 15°C. A quantitative analysis by HPLC showed that the reaction solution contained 2.45g of the target compound. Yield: 96.8% (production ratio A:B = 99.5:0.5)

### Example 11

To a mixture of 1.98g of 2-[4-(2,6-difluorophenyl)-thiazol-2-yl]-3-hydroxy-3-(2-trifluoromethylphenyl)-acrylonitrile and 15 ml of toluene were added 0.59g of triethylamine and 0.09g of γ-picoline. To the obtained mixture was added 0.94g of 2,2-dimethylpentanoyl chloride at 10 to 15°C. The resulting solution was stirred for 3 hours at 10 to 15°C. A quantitative analysis by HPLC showed that the reaction solution contained 2.53g of the target compound. Yield: 99.9% (production ratio A:B = 98.5:1.5)

### Comparative Example 1

Example 10 was repeated except that γ-picoline was not used. A quantitative analysis by HPLC showed that the reaction solution contained 1.75g of the target compound. Yield: 54.8% (production ratio A:B = 88:12)

### Example 12

To a mixture of 1.98g of 2-[4-(2,6-difluorophenyl)-thiazol-2-yl]-3-hydroxy-3-(2-trifluoromethylphenyl)-acrylonitrile, 15 ml of toluene, 5.5 ml of water and 0.20g of sodium hydroxide was added 0.94g of 2,2-dimethylpentanoyl chloride at a temperature range of 10 to 15°C, and then 0.30g of benzyl-n-butyl ammonium chloride (BTBAC) and 0.09g of γ-picoline were added at the same temperature. The resulting solution was stirred for 30 minutes at the same temperature. A quantitative analysis by HPLC showed that the reaction solution contained 2.45g of the target compound. Yield: 98.0% (production ratio A:B = 96.6:3.4)

### Example 13

To a mixture of 1.98g of 2-[4-(2,6-difluorophenyl)-thiazol-2-yl]-3-hydroxy-3-(2-trifluoromethylphenyl)-acrylonitrile, 15 ml of toluene and 5 ml of water were added 0.71 g of an aqueous solution of sodium hydroxide (containing 0.20g of NaOH) and 0.94g of 2,2-dimethylpentanoyl chloride at a temperature range of 10 to 15°C, and then 0.10g of triethylamine and 0.09g of γ-picoline were added at the same temperature. The resulting solution was stirred for an hour at the same temperature. A quantitative analysis by HPLC showed that the reaction solution contained 2.53g of the target compound. Yield: 100% (production ratio A:B = 98.2:1.8)

### Example 14

Example 13 was repeated except that tri-n-butylamine was used in place of triethylamine. A quantitative analysis by HPLC showed that the reaction solution contained 2.50g of the target compound. Yield: 99% (production ratio A:B = 99.4:0.6)

### Example 15

To a mixture of 0.69g of an aqueous solution of sodium hydroxide (containing 0.194g of NaOH), 0.045g of γ-picoline, 0.18g of tri-n-butylamine and 5 ml of chloroform was dropped a mixed solution of 1.98g of 2-[4-(2,6-difluorophenyl)-thiazol-2-yl]-3-hydroxy-3-(2-trifluoromethylphenyl)-acrylonitrile and 0.94g of 2,2-dimethylpentanoyl chloride in 10 ml of chloroform at a temperature range of 10 to 15°C. The resulting solution was stirred for 1.5 hours at the same temperature. A quantitative analysis by HPLC showed that the reaction solution contained 2.52g of the target compound. Yield: 99.9% (production ratio A:B = 99.8:0.2)

### Example 16

To a mixture of 0.27g of sodium hydroxide, 0.12g of γ-picoline and 0.27g of tri-n-butylamine in 20 ml of chloroform and 7 ml of water was dropped a mixed solution of 2.5g of 2-[4-(2,6-difluorophenyl)-thiazol-2-yl]-3-hydroxy-3-(2-trifluoromethylphenyl)-acrylonitrile and 1.28g of 2,2-dimethylpentanoyl chloride at a temperature range of 10 to 15°C. The resulting solution was stirred for an hour at the same temperature. A quantitative analysis by HPLC showed that the reaction solution contained 3.13g of the target compound. Yield: 98.1% (production ratio A:B = 99:1)

### Example 17

To a mixture of 0 98g of an aqueous solution of sodium hydroxide (containing 0.20g of NaOH), 0.023g of y-picoline, 0.45g of tri-n-butylamine and 10 ml of chloroform was dropped a mixed solution of 1.98g of 2-[4-(2,6-difluorophenyl)-thiazol-2-yl]-3-hydroxy-3-(2-trifluoromethylphenyl)-acrylonitrile and 0.94g of 2,2-dimethylpentanoyl chloride in 10 ml of chloroform at a temperature range of 10 to 15°C. The resulting solution was stirred for 1.5 hours at the same temperature. A quantitative analysis by HPLC showed that the reaction solution contained 2.52g of the target compound. Yield: 100% (production ratio A:B = 99.5:0.5)

### Applicability in Industry:

Use of the processes of the present invention makes it possible to produce O-acyl derivatives of compounds of enol form stereoselectively in a short time in high yield. These compounds are useful as intermediates for producing agrochemicals and drugs, and as final products. The processes of the present invention are valuable in industrial applications.

## Claims

1. A process for the preparation of an ethene derivative represented by Formula (I) 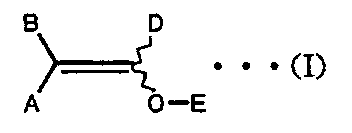 (wherein, A is optionally substituted hydrocarbon having 1 to 20 carbons, optionally substituted heterocyclic group, or a group represented by Formula R¹O, R¹S or R¹₁H₂₋₁N; R¹ is optionally substituted hydrocarbon having 1 to 20 carbons or optionally substituted heterocyclic group; 1 is 1 or 2; and when 1 is 2, R¹ may be the same or different; B is optionally substituted hydrocarbon having 1 to 20 carbons, optionally substituted heterocyclic group, CN, isonitrile, NO₂, N₃, CHO, or a group represented by Formula C(=X)R², S(O)mR² or P(=X)R²R³; R² and R³ are each independently optionally substituted heterocyclic group, optionally substituted hydrocarbon having 1 to 20 carbons, or a group represented by Formula R²⁰O, R²⁰S or R²⁰ₖH₂₋ₖN;R²⁰ is optionally substituted hydrocarbon having 1 to 20 carbons, or optionally substituted heterocyclic group; X is oxygen, sulfur, selenium, or a group represented by Formula NR²¹; R²¹ is optionally substituted hydrocarbon having 1 to 20 carbons, optionally substituted heterocyclic group, hydroxyl, or a group represented by Formula R²²O or R²²ₜH₂₋ₜN; R²² is optionally substituted hydrocarbon having 1 to 20 carbons, or optionally substituted heterocyclic group; m is 0, 1 or 2; k is 1 or 2; and when k is 2, R²⁰ may be the same or different; t is 1 or 2; and when t is 2, R²² may be the same or different; D is optionally substituted hydrocarbon having 1 to 20 carbons, or optionally substituted heterocyclic group; E is a group represented by Formula C(=X)R⁴, S(O)nR⁴ or P(=X)R⁴R⁵, alkoxymethyl having 1 to 6 carbons, alkylcarbonyloxymethyl having 1 to 6 carbons, cycloalkylcarbonyloxymethyl having 3 to 6 carbons, alkoxycarbonyloxymethyl having 1 to 6 carbons, optionally substituted phenylcarbonyloxymethyl, alkylthiomethyl having 1 to 6 carbons, alkylcarbonylthiomethyl having 1 to 6 carbons, cycloalkylcarbonylthiomethyl having 3 to 6 carbons, alkoxycarbonylthiomethyl having 1 to 6 carbons, optionally substituted phenylcarbonylthiomethyl, or optionally substituted phenylmethyl; R⁴ and R⁵ are each independently optionally substituted heterocyclic group, optionally substituted hydrocarbon having 1 to 20 carbons, or a group represented by Formula R⁴⁰O, R⁴⁰S or R⁴⁰_{q}H_{2-q}N; R⁴⁰ is optionally substituted hydrocarbon having 1 to 20 carbons, or optionally substituted heterocyclic group; X is oxygen, sulfur, selenium, or a group represented by Formula NR⁵¹; R⁵¹ is optionally substituted hydrocarbon having 1 to 20 carbons, or hydroxyl; n is 0, 1 or 2; q is 1 or 2; when q is 2, R⁴⁰ may be the same or different), **characterized in that** a compound represented by Formula (II) 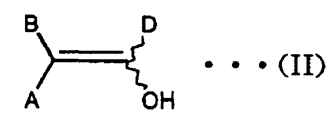 (wherein, A, B and D are as defined above) is reacted with a compound represented by Formula (III)
EY (III)
(wherein, E is as defined above; and Y is chlorine, bromine or CN; however, Y is chlorine or bromine when E is alkoxymethyl having 1 to 6 carbons, alkylcarbonyloxymethyl having 1 to 6 carbons, cycloalkylcarbonyloxymethyl having 3 to 6 carbons, alkoxycarbonyloxymethyl having 1 to 6 carbons, optionally substituted phenylcarbonyloxymethyl, alkylthiomethyl having 1 to 6 carbons, alkylcarbonylthiomethyl having 1 to 6 carbons, cycloalkylcarbonylthiomethyl having 3 to 6 carbons, alkoxycarbonylthiomethyl having 1 to 6 carbons, optionally substituted phenylcarbonylthiomethyl, or optionally substituted phenylmethyl) in the presence of a base and a pyridine derivative represented by Formula (IV) 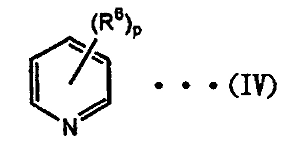 (wherein, R⁶ is hydrocarbon having 1 to 9 carbons; p is 0 or an integer of 1 to 3; and when p is 2 or more, R⁶ may be the same or different).

2. A process according to Claim 1, in which E is alkylcarbonyl having 1 to 10 carbons or (alkylthio)carbonyl.

3. A process according to Claim 1 or 2, in which A is optionally substituted phenyl, benzyl or aromatic heterocyclic group.

4. A process according to one of Claims 1 to 3, in which D is optionally substituted phenyl or pyrazolyl.

5. A process according to one of Claims 1 to 4, in which A, D and E are groups represented by the following formulae, respectively:
A: 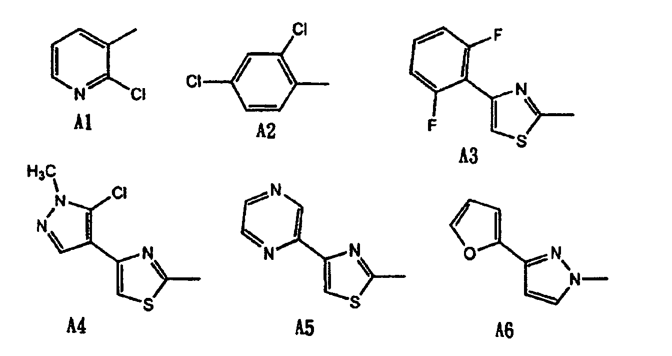 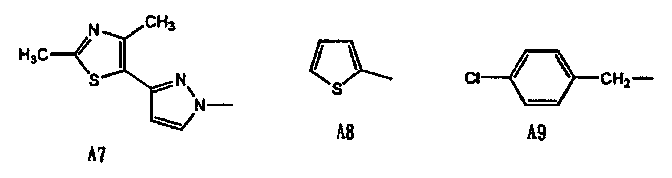
D: 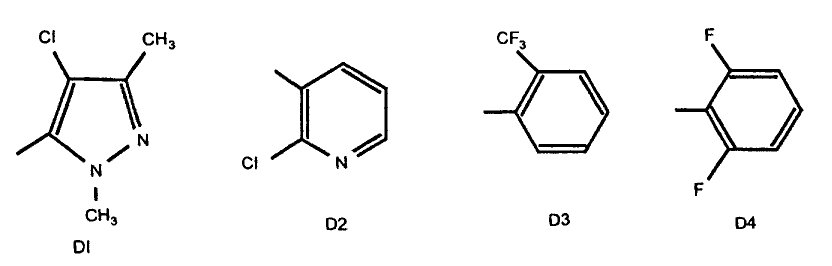 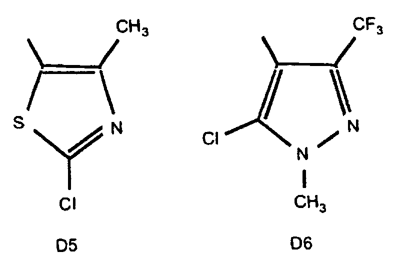
E:
| | | | | | |
|---|---|---|---|---|---|
| E1 | -COCH₃ | E2 | -COC(CH₃)₃ | E3 | -COSCH₃ |
| E4 | -COC(CH₃)₂-C₂H₅ | E5 | -COC(CH₃)₃-C₃H₇ | E6 | -CSCH₃ |

6. A process according to one of Claims 1 to 5, in which B is a CN group.

7. A process according to one of Claims 1 to 6, in which the reaction is carried out in an organic solvent

8. A process according to one of Claims 1 to 7, in which the base is a tertiary amine.

9. A process according to one of Claims 1 to 6, in which the reaction is carried out in a heterogeneous system of water and an organic solvent.

10. A process according to one of Claims 1 to 6 and 9, in which the base is an alkali metal hydroxide or a tertiary amine.

11. A process according to one of Claims 1 to 6, 9 and 10, in which the base is an alkali metal salt or a tertiary amine.

12. A process according to one of Claims 1 to 11, in which the pyridine derivative of Formula (IV) is lutidine or γ-picoline.
